Europäisches Patentamt

European Patent Office

Office européen des brevets

(11) Numéro de publication: **0 147 282**
A2

# (12) DEMANDE DE BREVET EUROPEEN

(21) Numéro de dépôt: **84402527.0**

(22) Date de dépôt: **07.12.84**

(51) Int. Cl.⁴: **A 61 K 7/48**

(30) Priorité: **09.12.83 FR 8319784**

(43) Date de publication de la demande:
**03.07.85 Bulletin 85/27**

(84) Etats contractants désignés:
**AT BE CH DE GB IT LI LU NL SE**

(71) Demandeur: **BIOSCULPTURE INTERNATIONAL**
**23 rue Duret**
**F-75116 Paris(FR)**

(72) Inventeur: **Lapinet, Engène Génia**
**1 Avenue du Commandant Guilbaud**
**F-75016 Paris(FR)**

(74) Mandataire: **Thibon-Littaye, Annick**
**Cabinet A. THIBON-LITTAYE 11 rue de l'Etang**
**F-78160 Marly-le-Roi(FR)**

(54) Compositions cosmétiques anti-surcharges graisseuses et application sur le corps humain par ionophorèse.

(57) La composition comprend un mélange de malates ainsi que de l'aminophylline, au moins un glycolysat choisi parmi les glycolysats de lierre grimpant, d'algues laminaires, de prêle, de bouleau ou de sarriette, du iodure de potassium et de la vitamine C.

Elle peut être appliquée sur le corps en soumettant la zone traitée à une ionisation par application d'un courant continu faible.

EP 0 147 282 A2

Croydon Printing Company Ltd

B 4076 F
TS/jl
DEC. 83

# COMPOSITIONS COSMETIQUES ANTI-SURCHARGES GRAISSEUSES ET APPLICATION SUR LE CORPS HUMAIN PAR IONOPHORESE

La présente invention concerne le domaine de la cosmétologie et les soins donnés à la peau.

Elle concerne plus particulièrement un nouveau produit destiné à remédier au phénomène de surcharges graisseuses, connu sous le nom de cellulite, et l'application de ce produit par ionophorèse.

Le phénomène de surcharges graisseuses, qui affecte principalement, mais non exclusivement, les femmes, est bien connu dans ses manifestations extérieures, mais cette expression générique, recouvre en fait trois catégories différentes de phénomènes.

Il y a en premier lieu les surcharges graisseuses constitutionnelles, de nature héréditaire et dont le traitement est tout à fait décevant. Il existe en outre les surcharges graisseuses acquises et celles liées à un excès de féminité, se manifestant par une simple surcharge graisseuse localisée.

De nombreuses solutions ont été avancées dans le passé pour remédier à cet état de fait.

On a déjà proposé, dans le cadre de traitements plus généraux du corps humain, de faire pénétrer des produits dans le corps à l'aide de dispositifs produisant des courants, la pénétration dans la peau se faisant alors par circulation d'ions.

On pourra se reporter plus particulièrement aux brevets FR 725 942, 726 625, 74 00385 et 75 39414.

La présente demande de brevet a pour objet d'apporter un perfectionnement à ces techniques, en proposant une nouvelle composition ionisable destinée à pénétrer dans la peau par la technique précédemment décrite qui est couramment dénommée "ionophorèse".

L'invention s'inspire en outre du brevet FR 82 01390 dans lequel on a décrit des compositions cosmétiques comprenant une association de malates et plus particulièrement de malates de métal bivalent, alcalino-terreux ou de transition et d'au moins un malate d'acide aminé basique.

La composition ionisable selon l'invention, pour application cutanée comprend un mélange de malates et elle est caractérisée en ce qu'elle comprend en outre de l'aminophylline, au moins un glycolysat, choisi parmi les glycolysats de lierre grimpant, d'algue laminaire, de prêle, de bouleau ou de sarriette, du iodure de potassium et de la vitamine C.

Les malates contenus dans la composition sont choisis parmi des malates de magnésium, de lysine, d'arginine et de silanol.

La composition peut comprendre en outre un agent d'élimination de l'eau tel que du mucopolysaccharide.

Il peut être souhaitable, lorsque la composition est préparée à l'avance, d'incorporer des agents de conservation connus en soi, tels que du propylène glycol, du parahydroxybenzoate de sodium et du chloracétamide.

La composition se présente le plus généralement sous forme d'une solution ou suspension aqueuse qui permet une application aisée comme on le décrira dans la suite de la présente description.

Les proportions des différents constituants de la composition peuvent varier dans de grandes proportions, l'essentiel étant la présence de ceux-ci.

On peut toutefois considérer que les proportions suivantes sont satisfaisantes, la composition étant prise sous sa forme la plus simple :

- de 0 à environ 70 % de malates,
- de 0 à environ 15 % d'aminophilline,
- de 0 à environ 10 % de iodure de potassium,

- de 0 à environ 30 % de glycolysat,
- de 0 à environ 5 % de vitamine C.

Lorsque la composition contient du propylène glycol ainsi que d'autres agents de conservation et du mucopolysaccharide, la composition peut être la suivante :

- de 0 à environ 35 % de malates,
- de 0 à environ 7 % en poids d'aminophilline,
- de 0 à environ 4 % en poids d'iodure de potassium,
- de 0 à environ 15 % en poids de glycolisat,
- de 0 à environ 2 % en poids de vitamine C,
- de 0 à environ 80 % en poids de propylène glycol,
- de 0 à environ 4 % en poids de paraoxybenzoate de méthyle sodium,
- de 0 à environ 2 % en poids de chloracétamide,
- de 0 à environ 7 % en poids de mucopolysaccharide.

Les pourcentages particulièrement préférés sont les suivants :

- d'environ 5 à environ 20 % en poids de malates,
- d'environ 1 à environ 3 % d'aminophilline,
- d'environ 1 à environ 2 % d'iodure de potassium,
- d'environ 5 à environ 8 % de glycolisat,
- d'environ 0,5 à environ 1 % de vitamine C,
- d'environ 60 à environ 70 % de propylène glycol,
- d'environ 1 à environ 2 % de paraoxybenzoate de méthyle sodium,

- d'environ 0,5 à environ 1 % de chloracétamide,
- d'environ 1,5 à environ 2,5 % de mucopolysaccharide.

En ce qui concerne les malates, il est approprié d'inclure une proportion prépondérante de malates de silanol, de l'ordre de 10 à 20 % et de compléter par environ 5 à 8 % de malate de magnésium, environ 0,1 à 0,5 % de malate de lisyne et environ 0,5 à environ 1 % de malate d'arginine.

Les glycolysats peuvent être quant à eux introduits en quantités égales, quand on utilise les glycolysats de lierre grimpant, d'algue laminaire, de prêle, de bouleau ou de sarriette.

La composition peut être préparée par simple mélange mais il est judicieux de dissoudre préalablement les malates dans la moitié du polypropylène glycol utilisé.

Aux fins de présentation, et pour permettre l'application sur le corps humain, la composition précédemment décrite, peut être dissoute dans l'eau dans des proportions variables. A titre d'exemple, il a été considéré qu'une solution comprenant de 10 à 20 % et plus particulièrement de l'ordre de 15 % en poids de compositions dans l'eau constituait une présentation satisfaisante.

L'application de la composition sur la peau se fait de la façon suivante.

Après que la surface cutanée ait été soigneusement nettoyée avec de l'eau et du savon, puis rincée, on pulvérise, à l'aide d'un dispositif connu en soi, une solution de la composition dans l'eau sur les endroits où apparaît la cellulite, par exemple sur le pourtour des cuisses ainsi que sur le pourtour de l'abdomen de part et d'autre de l'ombilic ou même sur le

B 4076 F
TS/jl
DEC. 83

0147282

visage.

On enveloppe ensuite le sujet à l'aide de bandes constituées par une imprégnation d'éponges végétales sur une trame textile, et ceci depuis les chevilles jusqu'au-dessus du pli de l'aine, ces bandes éponges ayant été préalablement trempées dans de l'eau tiède.

On dispose alors les bandes conductrices ou électrodes pour entourer les cuisses et l'abdomen du sujet en prenant la précaution de munir ce sujet d'une culotte ou d'un élément isolant en caoutchouc.

Les bandes conductrices ou électrodes sont alors mises sous une tension qui peut varier entre 0 et 90 volts et qui délivre une intensité de courant continu constant de l'ordre de 10 à 15 mA.

La durée du traitement est de l'ordre de 30 mn par séance.

Il est possible, soit au cours d'une même séance, soit lors de séances différentes, de prévoir une inversion de polarité, afin de changer le sens du courant.

Des essais ont révélés en premier lieu que la composition selon l'invention, non seulement n'exerçait aucun effet néfaste sur la peau mais qu'il apparaissait, après plusieurs séances du traitement précédemment décrit, une amélioration apparente de l'aspect présenté par la peau des patientes.

Sans vouloir être lié à une interprétation particulière, il est présumé que l'action de la composition selon l'invention est dûe aux points suivants :

- les malates doivent, au cours de la mise sous tension, libérer de l'acide malique sous l'effet de l'ionisation, l'acide malique entrant alors dans le cycle de KREBS et entraînant une carboxylation de l'acide pyruvique.

0147282

- l'arginine, quant à elle, doit participer à la synthèse de l'urée et donc à l'élimination de l'excès d'azote dans l'organisme, ce qui entraîne une désintoxication de la peau.

- la lysine, acide aminé essentiel aux propriétés biologiques importantes, favorise l'anabolisme azoté et augmente la protéinémie, elle exerce en outre une action hémopoiétique, joue un rôle dans le métabolisme du calcium, est hydroxylée in vivo, donne de l'hydroxyproline et donc concourt à la formation du collagène.

- le magnésium, quand il est utilisé sous la forme de son malate, joue un rôle important dans la plupart des métabolismes, rôle qui a été souligné par de nombreux auteurs. On peut noter d'ailleurs que la présence du magnésium est indispensable à l'adényl cyclase pour transformer l'ATP en AMP cyclique.

- le silanol introduit du silicium qui exerce une action amincissante.

On peut préciser que le malate de silanol également dénommé malate de monométhylsilanétriol, qui est en fait un complexe organique de silicium, a une action qui résulte avant tout du pontage des macromolécules du tissu conjonctif. Il semble intervenir dans la restructuration de ces tissus et est donc indispensable pour la synthèse par l'organisme de tissus conjonctifs sains.

On peut rappeler que

. pour CARLISLE E.M. (Siliconas essential element) Federation Proc. 33 1758-1766-1974), le silicium est un composé "intégral" des mucopolysaccharides. Agissant comme agent de pontage, il contribue à l'intégrité structurelle du tissu conjonctif.

pour KLAUS SCHWARTZ (A bound form of silicon in glycosaminoglycans and polyronides (polysaccharide matrix connective tissue) communicated by P.H. RAYER March 19, 1975, le silicium fonctionne comme un "cross-linking agent" (agent de réticulation) et contribue à l'architecture et à l'élasticité du tissu conjonctif.

Ainsi le malate de monométhylsilanétriol, en dehors de son action vectrice due à son pouvoir de pénétration élevé, a une action amincissante et un pouvoir de régénération tissulaire.

-la vitamine C aide à la transformation de la proline en hydroxyproline, seule protéine tissulaire présente dans le collagène et l'élastine.

- La caractéristique essentielle des glycolysats de plantes est la présence de flavanoïdes, décelable par chromatographie, et dont la disposition permet de caractériser la plante à laquelle on a affaire. Il y a donc dans ces glycolysats une présence effective des différentes flavanoïdes (surtout quercitine, rutine, etc.). Or ces flavanoïdes, en dehors du renforcement de la paroi des capillaires, possèdent une action anti-inflammatoire (même préventive), et une action anti-allergique. Ces actions se surajoutent à l'action de la plante elle-même, comme par exemple l'effet amincissant des algues laminaires ou l'action anti-bactérienne de la sarriette.

- enfin, les mucopolysaccharides favorisent l'élimination de l'eau et des toxines et contribuent à la revascularisation de l'épiderme.

- dans la présente description tous les pourcentages ont été exprimés en poids.

L'exemple ci-après est destiné à illustrer l'invention sans aucunement en limiter la portée.

On a réalisé une composition selon l'invention en milieu aqueux ayant la formule pondérale suivante :

| | |
|---|---|
| Aminophylline | 3 g |
| Iodure de potassium | 2 g |
| Glycolysat lierre grimpant | 2 g |
| Glycolysat algues laminaires | 2 g |
| Glycolysat de prêle | 2 g |
| Glycolysat de bouleau | 2 g |
| Glycolysat de sarriette | 2 g |
| Malate de magnésium | 8,5 g |
| Malate de lysine | 0,22 g |
| Malate d'arginine | 1 g |
| Propylène glycol | 90.28 |
| Paraoxybenzoate de méthyl de Na | 2 g |
| Chloracétamide | 1 g |
| Mucopolysaccharide | 3 g |
| Vitamine C | 1 g |
| Malate de silanol | 20 g |
| eau qsp | 1000 |

En fait, cette composition avait été réalisée à partir d'une dissolution de 19,44 g d'une solution du mélange des malates de magnésium lysine et arginine dans 50 % de propylène glycol.

Le malate de monométhylsilanétriol était en solution à 1 %, seule dissolution lui permettant d'être stable.

Composition analytique : %

| | |
|---|---|
| monométhylsilanétriol | 41,22 g |
| silicium | 12 g |
| acide malique | 58,77 g |

Spécification :

liquide légèrement opalescent

B 4076 F
TS/jl
DEC. 83

pH                                    de 4 à 5,5

densité                               0,990

miscible à l'eau

insoluble dans les solvants organiques : alcools,
glycols etc.

10 ml de cette solution ont été pulvérisés sur une patiente dont la peau avait été préalablement soigneusement lavée au savon et rincée.

Après cette pulvérisation, on a entouré le corps de la patiente par des bandes constituées par une enduction d'éponge végétale sur une trame en coton et qui avaient été préalablement imbibées d'eau tiède ou légèrement chaude et essorées.

Puis on a disposé sur les parties du corps de la patiente comportant des surcharges graisseuses, des plaques conductrices ou électrodes qui étaient reliées à un générateur spécial de courant galvanique à multicanaux distribuant un courant continu constant de 90 volts sous une intensité de l'ordre de 12 mA.

Cet appareil était bien entendu muni des sécurités requises et notamment d'une prise à la terre et d'une protection par fusible rapide.

On a marqué le passage du courant pendant 30 mn.

Cette expérience a été renouvelée une dizaine de fois à trois ou quatre jours d'intervalles.

Au bout de ces séances, il a été considéré par les observateurs et par la patiente que l'aspect de la peau qui avait été ainsi traitée était considérablement amélioré semblant révéler une diminution au moins apparente des surcharges graisseuses.

On a procédé à des essais de tolérance cutanée chez le lapin.

L'essai a été réalisé sur six lapins mâles Albinos néo-zélandais, préalablement épilés selon la

B 4076 F
TS/jl
DEC. 83

0147282

méthode décrite dans l'arrêté du 5 avril 1971, modifié le 21 février 1982 et concernant les techniques d'irritation primaire cutanée.

Chaque lapin a reçu deux applications du produit, à savoir :

- 0,5 ml de produit pur et plus particulièrement une sur la peau scarifiée avec un vaccinostyle, une sur la peau intacte (environ 2 $cm^2$)

On a ainsi déterminé l'indice d'irritation primaire cutanée :

- en notant l'érythème de 0 à 4,
- en notant l'oedème de 0 à 4.

Le produit est considéré comme :

| | |
|---|---|
| - non irritant pour un indice | 0,5 |
| - légèrement irritant pour un indice compris entre | 0,5 et 2 |
| - irritant pour un indice compris entre | 2 et 5 |
| - très irritant pour un indice compris entre | 5 et 8 |

Les résultats expérimentaux sont réunis dans le Tableau ci-après.

Il va de soi que l'exemple de réalisation de la composition selon l'invention qui a été précédemment donné ne constitue qu'une des possibilités et il serait possible d'ajouter des constituants complémentaires qui rentrent dans la définition de l'invention (malates notamment) ou qui constituent des adjuvants complémentaires connus permettant de traiter la peau.

Pour ce qui concerne la mise en oeuvre du procédé selon l'invention, qui consiste à appliquer la composition précédemment décrite en soumettant la zone cutanée traitée à une ionisation par application d'une courant continu faible à polarité temporairement inversible inférieur à 40 volts et sous une intensité ne dépassant pas quelques milli-ampères, on pourra se reporter au brevet FR 75 39414.

On notera en outre qu'il est possible de compléter l'application de la mise en oeuvre du procédé selon l'invention par le dépôt sur la peau traitée d'une crème, constituée à partir des complexes de malate précédemment évoqués et ayant par exemple la composition suivante :

- stéarine de 2 à 5 % de préférence 3,5 %
- émulcire de 3 à 7 % de préférence 5,5 %
- crodamol pmp de 6 à 10 % de préférence 8 %
- propylène glycol de 3 à 7 % préférence 5 %
- cétiol V de 2 à 5 % de préférence 3,5 %
- céphène de 0 à 1 % de préférence 0,6 %
- paraoxybenzoate de méthyle de 0 à 0,5% de préférence de 0,2 %
- paraoxybenzoate de propyle de 0 à 0,2 % de préférence 0,1 %
- paraoxybenzoate de méthyle sodé de 0 à 0,2 % de préférence 0,1 %
- B.H.T. 0,1 %
- verveine transcuta 0,1 %
- thiophylline de 0 à 0,6 % de préférence 0,4 %
- glycolysat algues laminaires de 0 à 0,4 % de préférence 0,2 %
- glycolysat lierre grimpant de 0 à 0,5 % de préférence de 0,3 %
- glycolysat de sauge de 0 à 0,4 % de préférence 0,2 %
- complexe de malates 0,2 %
- eau distillée complément à 100 %, par exemple 71,30 % pour la composition optimum.

## TEST D'IRRITATION CUTANEE

| lapins | | 24 HEURES | | 72 HEURES | | TOTAL |
|---|---|---|---|---|---|---|
| | | Erythème | Oedème | Erythème | Oedème | Er+Oe |
| 1 | normale | 0 | 0 | 0 | 0 | 0 |
| | scarifiée | 0 | 0 | 0 | 0 | 0 |
| 2 | normale | 0 | 0 | 0 | 0 | 0 |
| | scarifiée | 0 | 0 | 0 | 0 | 0 |
| 3 | normale | 0 | 0 | 0 | 0 | 0 |
| | scarifiée | 0 | 0 | 0 | 0 | 0 |
| 4 | normale | 1 | 0 | 0 | 0 | 1 |
| | scarifiée | 1 | 0 | 0 | 0 | 1 |
| 5 | normale | 0 | 0 | 0 | 0 | 0 |
| | scarifiée | 0 | 0 | 0 | 0 | 0 |
| 6 | normale | 0 | 0 | 0 | 0 | 0 |
| | scarifiée | 0 | 0 | 0 | 0 | 0 |
| | | | | | total | 2 |

INDICE $\dfrac{T}{24}$

RESULTAT : 0,08

B 4076 F
TS/jl
DEC. 83

# REVENDICATIONS

1.    Composition ionisable pour application cutanée comprenant un mélange de malates, caractérisée en ce qu'elle comprend en outre de l'aminophylline, au moins un glycolysat choisi parmi les glycolysats de lierre grimpant, d'algues luminaires, de prêle, de bouleau ou de sarriette, du iodure de potassium et de la vitamine C.

2.    Composition selon la revendication 1, caractérisée en ce que les malates sont choisis parmi les malates de magnésium, de lysine, d'arginine et de silanol.

3.    Composition selon l'une des revendications 1 ou 2, caractérisée en ce qu'elle contient en outre au moins un agent d'élimination de l'eau tel que du mucopolysaccharide.

4.    Composition selon l'une quelconque des revendications 1 à 3, caractérisée en ce qu'elle contient des agents de conservation tels que du propylène glycol, du parahydroxybenzoate de sodium et du chloracétamide.

5    Composition selon l'une quelconque des revendications 1 à 2, caractérisée en ce qu'elle comprend :

- de 0 à 70 % de malates
- de 0 à 15 % d'aminopylline
- de 0 à 10 % d'iodure de potassium
- de 0 à 30 % de glycolysats
- de 0 à 5 % de vitamine C.

6.    Composition selon l'une des revendications 3 ou 4, caractérisée en ce qu'elle contient :

- de 0 à 35 % de malates
- de 0 à 7 % d'aminophylline
- de 0 à 4 % d'iodure de potassium
- de 0 à 15 % de glycolysats
- de 0 à 2 % de vitamine C
- de 0 à 80 % de propylène glycol

4076 F
TS/jl
EC. 83

- de 0 à 4 % de paraoxybenzoate de méthyle
  sodium

- de 0 à 2 % de chloracétamide

- de 0 à 7 % de muccopolysaccharide.

7.      Composition selon la revendication 5, caractérisée en ce qu'elle contient :

| | |
|---|---|
| Aminophylline | 3 g |
| Iodure de potassium | 2 g |
| Glycolysat de lierre grimpant | 2 g |
| Glycolysat d'algues laminaires | 2 g |
| Glycolysat de prêle | 2 g |
| Glycolysat de bouleau | 2 g |
| Glycolysat de sarriette | 2 g |
| Malate de magnésium | 8,5 g |
| Malate de lysine | 0,22 g |
| Malate d'arginine | 1 g |
| Propylène glycol | 90.28 |
| Paraoxybenzoate de méthyl de Na | 2 g |
| Chloracétamide | 1 g |
| Mucopolysaccharide | 3 g |
| Vitamine C | 1 g |
| Malate de silanol | 20 g |
| eau qsp | 1000 g |